**Europäisches Patentamt**

**European Patent Office**

**Office européen des brevets**

(19)

(11) Publication number : **0 416 855 B1**

## (12) EUROPEAN PATENT SPECIFICATION

(45) Date of publication of patent specification :
08.12.93 Bulletin 93/49

(51) Int. Cl.⁵ : **A61K 31/20**

(21) Application number : **90309638.6**

(22) Date of filing : **04.09.90**

(54) **Fatty acids for the treatment and prevention of skin damage caused by radiotherapy.**

(30) Priority : **07.09.89 GB 8920228**

(43) Date of publication of application :
**13.03.91 Bulletin 91/11**

(45) Publication of the grant of the patent :
**08.12.93 Bulletin 93/49**

(84) Designated Contracting States :
**AT BE CH DE DK FR GB GR IT LI LU NL SE**

(56) References cited :
**EP-A- 0 101 294**
**EP-A- 0 115 419**
**EP-A- 0 275 643**
**EP-A- 0 309 086**
**EP-A- 0 334 507**

(56) References cited :
**An. Real Acad. Farm. vol. 51, 1985, pp. 327-332;**
**J. Valladares et al.**
**Z. Hautkr, vol. 62, suppl. 1, 30th June 1987, pp.**
**100-103, Grosse Verlag, Berlin, DE; W. Meigel**
**et al.**

(73) Proprietor : **SCOTIA HOLDINGS PLC**
**Efamol House Woodbridge Meadows**
**Guildford Surrey GU1 1BA (GB)**

(72) Inventor : **Horrobin, David Frederick**
**c/o Efamol House, Woodbridge Meadows**
**Guildford, Surrey GU1 1BA (GB)**
Inventor : **Scott, Catherine Ann**
**c/o Efamol House, Woodbridge Meadows**
**Guildford, Surrey GU1 1BA (GB)**

(74) Representative : **Miller, Joseph et al**
**J. MILLER & CO. 34 Bedford Row, Holborn**
**London WC1R 4JH (GB)**

**Description**

FIELD OF INVENTION

The invention relates to fatty acid therapy and compositions therefor.

BACKGROUND

Radio-therapy is a major treatment modality in cancer. However, radiation damages normal tissues as well as cancerous tissues and this damage is a major limitation in its use. Since radiation has to pass through the skin to reach the cancer, skin damage in particular can be an important problem. Such damage can be minimised by careful attention to radiation dosage, radiation direction and radiation focusing but it can never entirely be eliminated. The skin damage leads to burning, pain, itching, inflammation and sometimes loss of the surface layers of the skin. If it could be reduced, prevented or reversed, a major therapeutic advance would have been made.

Ionising radiation can destroy essential fatty acids (EFAs) by attacking the double bonds which are essential for their EFA activity. Many years ago ionising radiation was shown to be much more destructive in animals which were EFA-deficient and also to induce signs of EFA deficiency (Decker et al, J. Nutrition 41: 507, 1950; Cheng et al, J. Nutrition 48: 161 to 82, 1952). It is therefore possible that some of the damage to tissues by radiation may relate to loss of essential fatty acids. This could be particularly true of the skin, since EFAs are exceptionally important to skin function.

In Z. Hautkr., 62 (suppl.1)(1987), 100-103 it is disclosed that high doses of LA and GLA can produce decreases in eczema-related skin changes in patients suffering from atopical dermatitis.

EP 115 419 is concerned with the treatment of atopic disorders such as eczema, asthma, allergies, migraine and other disorders associated with atopy, by administering to the sufferer one or more metabolites of linoleic acid, together with one or more metabolites of $\alpha$-linolenic acid.

The main EFA in the diet is linoleic acid (LA), but in order to be fully effective as an EFA this acid must be converted to gamma-linolenic acid (GLA). This conversion cannot take place in the skin itself, but must be carried out in other tissues, notably the liver, from which the GLA and its metabolites can be transported to the skin. For optimal health the skin needs both linoleic acid on the one hand and GLA and its metabolites on the other.

THE INVENTION

We propose that much of the skin damage caused by radio-therapy is related to loss of EFAs from the skin, and also that the skin damage may be reduced or prevented by administration of appropriate EFAs. Linoleic acid alone or GLA alone is effective but optimum results are obtained by providing both linoleic acid and GLA. Dihomo-gamma-linolenic acid (DGLA) to which GLA is converted in the body has the same effect as GLA and references to the use of GLA herein may be taken as references to the use of DGLA with or instead of it.

The invention thus lies in the use of LA or GLA/DGLA or both for prevention or reversal of radiation induced skin damage, and to the use of LA or GLA/DGLA in the preparation of medicaments for such purposes wherein such acids are used.

Amounts of the acids are in the case of linoleic acid 0.1 to 100 g per day, preferably 1 to 20 g per day, very preferably 2 to 10 g per day, and in the case of GLA/DGLA 0.01 to 100 g per day, preferably 0.1 g to 10 g per day, very preferably 0.3 to 2.0 g per day.

Administration may be systemic or topical, and in the latter case the fatty acids are applied to the area which is to be or is being irradiated as such or in any appropriate vehicle. The vehicle may be the native oils in which the fatty acids are found, or such oils or the fatty acids themselves in any appropriate chemical form formulated as creams, gels, lotions, ointments or any other suitable format. The concentrations of the fatty acids in the topical preparation may range from 0.01 to 100%, preferably 0.1 to 20% and very preferably 1 to 10%.

EFAs GENERALLY

The fatty acids used according to the invention are related to other EFAs as follows:-

2

| n-6 | n-3 |
|---|---|
| 18:2 delta-9,12<br>(linoleic acid) | 18:3 delta-9,12,15<br>(alpha-linolenic acid) |

delta-6 desaturase

| 18:3 delta-6,9,12<br>(gamma-linolenic acid) | 18:4 delta-6,9,12,15 |

elongation

| 20:3 delta-8,11,14<br>(dihomo-gamma-linolenic acid) | 20:4 delta-8,11,14,17 |

delta-5 desaturase

| 20:4 delta-5,8,11,14<br>(arachidonic acid) | 20:5 delta-5,8,11,14,17 |

elongation

| 22:4 delta-7,10,13,16<br>(adrenic acid) | 22:5 delta-7,10,13,16,19 |

delta-4 desaturase

| 22:5 delta-4,7,10,13,16 | 22:6 delta-4,7,10,13,16,19 |

The pathways are not normally reversible nor, in man, are n-3 and n-6 series acids inter-convertible.

The acids, which naturally are of the all-cis configuration, are systematically named as derivatives of the corresponding octadecanoic, eicosanoic or docosanoic acids, e.g. delta-9,12-octadecadienoic acid or delta-4,7,10,13,16,19 docosahexaenoic acid, but numerical designation such as, correspondingly, 18:2 n-6 or 22:6 n-3 is convenient. Initials, for example, DHA for 22:6 n-3 (docosahexaenoic acid), are also used but do not serve when n-3 and n-6 acids of the same chain length and degree of unsaturation exist. Trivial names in more or less common use in the n-6 series are as shown. Of the n-3 series only 18:3 n-3 has a commonly used trivial name, alpha-linolenic acid. It was characterised earlier than gamma-linolenic acid and reference in the literature simply to linolenic acid, especially in the earlier literature, is to the alpha-acid.

DERIVATIVES

The acids may be used as such or as pharmaceutically acceptable and physiologically equivalent derivatives as, for example, detailed later herein for GLA and DGLA, and reference to any of the acids is to be taken as including reference to the acids when in the form of such derivatives. Equivalence is demonstrated by entry into the pathway quoted herein, as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al, page 23, "Analysis of Lipids and Lipoproteins" Ed Perkins, American Oil Chemists Society, Champaign, Illinois, U.S.A.

In outline the method is suitably that plasma samples (1 ml) are extracted with chloroform:methanol (2:1). The extract is filtered through sodium sulphate, evaporated to dryness, and taken up in 0.5 ml chloroform:methanol. The lipid fractions are separated by thin layer chromatography or silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot (183 cm) column packed with 10% silar on chromosorb WAW 106/230. The carrier gas is helium (30 ml/min). Oven temperature is programmed to rise from 165°C to 190°C at 2°C/min. Detector temperature is 220°C and injector temperature 200°C. Retention times and peak areas are automatically computed by Hewlett-Packard Level 4 integrator. Peaks are identified by comparison with standard fatty acid methyl esters.

## DIETARY COMPOSITIONS

The invention is chiefly described in terms of methods of treatment and pharmaceutical compositions, but it will be understood that the gamma-linolenic and other acids, being in the nature of dietary supplements, can be incorporated in a dietary margarine or other foodstuffs and such are to be understood as within the term pharmaceutical composition when for the purposes set out.

## FORMS AND SOURCES OF GAMMA-LINOLENIC AND OTHER ACIDS

Convenient physiologically equivalent derivatives of gamma-linolenic acid and dihomo-gamma-linolenic acid for use according to the invention as with the other acids, includes salts, amides, esters including glyceride esters and alkyl (e.g. $C_1$ to $C_4$) esters, and phospholipids.

If desired, pharmaceutical compositions may be produced for use in the invention by associating the natural or synthetic acids, as such or as derivatives, with an acceptable pharmaceutical vehicle. It is, however, at present convenient to provide at least the gamma-linolenic acid in the form of an available oil having a high gamma-linolenic acid content, hence reference to "oil" herein.

At the present time known natural sources of oils having a high gamma-linolenic acid content are few. One source of oils currently available is, however, the seed of Evening Primrose species such as <u>Oenothera biennis L.</u> and <u>Oenothera Lamarckiana</u>, the oil extract therefrom containing gamma-linolenic acid (about 8%) and linoleic acid (about 72%) in the form of their glycerides, together with other glycerides (percentages based on total fatty acids). Other sources of gamma-linolenic acids are Borage species such as <u>Borago officinalis</u> which, though current yield per acre is low, provide a richer source of gamma-linolenic acid than Oenothera oil.

The oil is extracted from the seed by one of the conventional methods of extraction such as cold pressure, screw pressure after partially cooking the seed, or solvent extraction.

Fractionation of a typical sample of Evening Primrose seed oil in the form of methyl esters shows the relative proportions:

| Palmitate | 6.15 |
| Stearate | 1.6 |
| Oleate | 10.15 |
| Linoleate | 72.6 |
| Gamma-linolenate | 8.9 |

The seed oil extracts referred to above can be used as such or can, for example, if desired, be fractionated to yield an oily composition containing the tri-glycerides of gamma-linolenic and linoleic acids as the main fatty acid components, the gamma-linolenic acid content being, if desired, a major proportion. Seed oil extracts appear to have a stabilising effect upon dihomo-gamma-linolenic acid if present.

Recent studies on fungi which can be cultivated by fermentation promise a fungal oil sources of both gamma-linolenic and dihomo-gamma-linolenic acids.

## PHARMACEUTICAL PRESENTATION

The compositions are conveniently in a form suitable for systemic (e.g. oral, rectal or parenteral) administration in a suitable pharmaceutical vehicle, as discussed in detail, for example, in Williams British Patent Specification No. 1,082,624, to which reference may be made, and in any case very well known generally for any particular kind of preparation. Thus, for example, tablets, capsules, ingestible liquid or powder preparations can be prepared as required, and topical preparations also when the gamma-linolenic acid or other acids are absorbed through the skin. Injectable solutions of hydrolysed Oenothera oil may be prepared using albumin to solubilise the free acid.

Other forms are the topical oils, creams, etc. referred to earlier herein, also very well known generally in themselves.

Advantageously, a preservative is incorporated into the preparation. Alpha-tocopherol in concentration of about 0.1% by weight has been found suitable for the purpose.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

Ideally treatment should start one or two weeks before radio-therapy begins and should continue for several weeks after it ends. Less complete but still valuable protection will be afforded by shorter periods of treatment.

## EXAMPLES OF COMPOSITIONS

The following compositions are administered to treat or prevent skin damage in patients undergoing radio-therapy:-
(1) 3 to 6 capsules twice per day of 500 mg capsules of evening primrose oil.
(2) 3 to 6 capsules twice per day of 500 mg capsules of blackcurrant oil.
(3) 3 to 6 capsules twice per day of 500 mg capsules of microbial oil from the species Mortierella alpina, rich in GLA.
(4) 3 to 6 capsules twice per day of 500 mg capsules of borage oil.
(5) 5 to 15 capsules per day of 500 mg capsules of safflower oil, corn oil or sunflower oil, as a source of LA, in glyceride form, without GLA.
(6) 4 capsules per day, each containing 250 mg pure GLA or DGLA.
(7) 10 capsules per day, each containing 500 mg pure LA.
(8) A cream containing 15% evening primrose oil providing approximately 1.2% GLA and 10.5% LA.
(9) An ointment containing 2% of ethyl gamma-linolenate.
10. An ointment containing 1% of ethyl gamma-linolenate and 5% of ethyl linoleate.

## CASE HISTORIES

Two specific examples of case histories, one curative the other preventive of skin damage, are given:-
(i) A thirty seven year old woman developed a lump in her left breast which biopsy showed to be due to cancer. The lump was removed surgically and a course of radio-therapy initiated. The skin became severely inflamed and after three weeks, had broken down over an extensive area. The woman took 500 mg capsules, 8 per day, of evening primrose oil, containing both LA and GLA. Within fourteen days there was considerable improvement in her skin condition, in spite of continuation of the radio-therapy. The skin surface healed, and the inflammation, pain and irritation were much reduced.
(ii) A forty nine year old woman developed a lump in her right breast which was found to be cancerous and was surgically removed as was a lymph node in the axilla. A course of radio-therapy followed. Two weeks before starting the radio-therapy, the woman started taking 6 x 500 mg capsules of evening primrose oil per day. The expected skin damage was dramatically milder than expected and amounted to the equivalent of little more than a mild sunburn. The skin remained healthy during the whole nine weeks of the radio-therapy course.

These two case histories show that administration of EFAs can both reverse and prevent skin damage due to radio-therapy. Both women experienced a considerable improvement in overall well being so that it cannot be excluded that systemic ill effects of radio-therapy are also reduced.

## Claims

### Claims for the following Contracting States : AT, BE, DE, DK, FR, GB, IT, LI, LU, NL, SE

1. Use of (a) linoleic acid (LA), or (b) gamma-linolenic acid (GLA) or dihomo-gamma-linolenic acid (DGLA), or (c) both said LA and said GLA or DGLA, or pharmaceutically acceptable and physiologically equivalent derivatives of the acids in the preparation of a medicament for treating or preventing skin damage in patients undergoing radiotherapy.

2. A use according to claim 1 wherein said medicament is in a form for systemic administration and comprises linoleic acid in a dose range of 0.1 to 100 g per day, preferably 1 to 20 g per day, very preferably 2 to 10 g per day, or GLA or DGLA suitable for administration in a dose range of 0.01 to 100 g per day, preferably 0.1 to 10 g per day, very preferably 0.3 to 2.0 g per day, or both said LA and said GLA or DGLA.

3. A use according to claim 1 wherein the medicament is in a form for topical administration and comprises the fatty acid(s) in a total concentration of 0.01 to 100%, preferably 0.1 to 20%, and very preferably 1 to 10% of the composition, balance of the composition being a pharmaceutically acceptable diluent or carrier.

### Claim for the following Contracting State : GR

1. Use of (a) linoleic acid (LA), or (b) gamma-linolenic acid (GLA), or dihomo-gamma-linolenic acid (DGLA), or (c) both said LA and said GLA or DGLA, or pharmaceutically acceptable and physiologically equivalent derivatives of the acids in the preparation of a medicament for treating or preventing skin damage in patients undergoing radiotherapy.

## Patentansprüche

### Patentansprüche für folgende Vertragsstaaten : AT, BE, DE, DK, FR, GB, IT, LI, LU, NL, SE

1. Verwendung (a) einer Linolensäure (LA) oder (b) einer Gamma-Linolensäure (GLA) oder Dihomogammalinolensäure (DGLA) oder (c) sowohl der besagten LA und der besagten GLA oder DGLA, oder von pharmazeutisch akzeptablen und physiologisch gleichwertigen Derivaten der Säuren zur Präparation eines Medikamentes für die Behandlung oder Vorbeugung von Hautschäden bei Strahlentherapiepatienten.

2. Eine Verwendung gemäß Anspruch 1, bei welcher das besagte Medikament in einer Form zur systemischen Verabreichung vorliegt und aus Linolensäure in einem Dosisbereich von 0,1 bis 100 g pro Tag, vorzugsweise 1 bis 20 g pro Tag, noch besser 2 bis 10 g pro Tag, oder GLA oder DGLA, die zur Verabreichung in einem Dosisbereich von 0,01 bis 100 g pro Tag, vorzugsweise 0,1 bis 10 g pro Tag, noch besser 0,3 bis 2,0 pro Tag, geeignet sind, oder sowohl aus besagter LA und besagter GLA oder DGLA besteht.

3. Eine Verwendung gemäß Anspruch 1, bei welcher das Medikament in einer Form zur äußeren Anwendung vorliegt und die Fettsäure(n) in einer Gesamtkonzentration von 0,01 bis 100%, vorzugsweise 0,1 bis 20%, und noch besser 1 bis 10% der Zusammensetzung enthält, wobei der Rest der Zusammensetzung ein pharmazeutisch akzeptabler Verdünner oder Träger ist.

### Patentanspruch für folgenden Vertragsstaat : GR

1. Verwendung (a) einer Linolensäure (LA) oder (b) einer Gamma-Linolensäure (GLA) oder Dihomogammalinolensäure (DGLA) oder (c) sowohl der besagten LA und der besagten GLA oder DGLA, oder von pharmazeutisch akzeptablen und physiologisch gleichwertigen Derivaten der Säuren zur Präparation eines Medikamentes für die Behandlung oder Vorbeugung von Hautschäden bei Strahlentherapiepatienten.

## Revendications

### Revendications pour les Etats contractants suivants : AT, BE, DE, DK, FR, GB, IT, LI, LU, NL, SE

1. Utilisation de (a) l'acide linoléïque (AL) ou (b) de l'acide gamma-linolénique (AGL) ou de l'acide dihomo-gamma-linolénique (ADGL) ou (c) à la fois ledit AL et ledit AGL ou ledit ADGL, ou de dérivés pharmaceutiquement acceptables et physiologiquement équivalents desdits acides dans la préparation d'un médicament pour le traitement ou la prévention des lésions cutanées chez des patients soumis à une radiothérapie.

2. Utilisation selon la revendication 1, dans laquelle ledit médicament est sous une forme destinée à l'administration systémique et comprend de l'acide linoléïque dans un intervalle de doses compris entre 0,1 et 100 g par jour, de préférence entre 1 et 20 g par jour, de façon encore plus préférée entre 2 et 10 g par jour, ou de l'AGL ou de l'ADGL convenant pour l'administration dans un intervalle de doses compris entre 0,01 et 100 g par jour, de préférence entre 0,1 et 10 g par jour, de façon encore plus préférée entre 0,3 et 2,0 g par jour, ou à la fois ledit AL et ledit AGL ou ADGL.

3. Utilisation selon la revendication 1, dans laquelle le médicament est sous une forme destinée à l'administration topique et comprend le(s) acide(s) gras à une concentration totale de 0,01 à 100 %, de préférence de 0,1 à 20 %, et de façon encore plus préférée de 1 à 10 % de la composition, le reste de la composition étant un diluant ou un support pharmaceutiquement acceptable.

### Revendication pour l'Etat contractant suivant : GR

1. Emploi (a) d'un acide linoléique (AL), ou (b) d'un acide gamma-linolénique (AGL), ou d'un acide dihomo-gamma-linolénique (ADGL) ou (c) à la fois dudit AL et dudit AGL ou dudit ADGL, ou de dérivés équivalents, acceptables des points de vue pharmaceutique et physiologique des acides, dans la préparation d'un médicament servant au traitement ou la prévention des détériorations de la peau chez des patients traités par radiothérapie.